# EUROPÄISCHE PATENTANMELDUNG

(11) **EP 1 357 178 A1**
(43) Veröffentlichungstag der Anmeldung: **29.10.2003**
(21) Anmeldenummer: 02008966.0
(22) Anmeldetag: 22.04.2002
(51) Int. Cl.: C12M 3/00, B01L 3/00

(54) **Mikrofluidsystem**

(71) Anmelder: ibidi GmbH, 80799 München (DE)
(72) Erfinder: Kahl, Johan-Valentin Dr, 80636 München (DE)
(74) Vertreter: Grünecker, Kinkeldey, Stockmair & Schwanhäusser Anwaltssozietät

(57) **Zusammenfassung**

Die Erfindung betrifft ein Mikrofluidsystem mit einem Substrat, wenigstens zwei Reservoiren und wenigstens einem in das Substrat integrierten Kanal, der zwei Reservoire verbindet, wobei die Mündung wenigstens eines Kanals oberhalb der Grundfläche eines Reservoirs angeordnet ist.

## Beschreibung

Die Erfindung betrifft ein Mikrofluidsystem mit einem Substrat, wenigstens zwei Reservoiren und wenigstens einem in das Substrat integrierten Kanal, der zwei Reservoire verbindet.

Viele biologischen/chemischen Analysen werden mittels (mikro)-fluidischer Komponenten durchgeführt. Diese zeichnen sich durch einfache oder vernetzte Kanalsysteme aus. Um die Flüssigkeiten durch solche Kanäle zu leiten, werden Pumpen verwendet. Dies ist besonders dann nötig, wenn viel Flüssigkeit durch ein Kanalsystem geführt werden soll, oder wenn ein und dieselbe Flüssigkeit wiederholt durch das Kanalsystem geleitet werden soll.

Die Pumpen können im oder am mikrofluidischen System angebracht sein. Pumpen haben jedoch viele Nachteile. So kann das Mikrofluidsystem nur in Verbindung mit einer externen Energiequelle verwendet werden und muß an dieses fest angeschlossen werden. (Bei einer integrierten Pumpe ist ein Stromkabel erforderlich, bei einer externen Pumpe muß das System mit Schläuchen verbunden werden).

Kommen Komponenten der Pumpe in Kontakt mit der Probenlösung, kann es zu chemisch nicht erwünschten Reaktionen kommen. Durch Verwendung von Pumpen besteht ein großes Totvolumen, welches insbesondere beim Austausch von Lösungen zu Problemen bei der Verwendung von wenig Probenmaterial führt. Viele Pumpen pulsieren, was zu starken Druckschwankungen im Kanal führt. Dies kann zu Beschädigungen gerade bei kleinen Kanälen führen. Auch führen selbst kleine Pumpen zu großen Strömungsgeschwindigkeiten in einem verhältnismäßig kleinen Kanalsystem. Dadurch können an der Kanaloberfläche fixierte Moleküle oder Zellen abgelöst oder beschädigt werden.

Der kontinuierliche oder zyklische Lösungswechsel in einem Kanalsystem ist für viele Anwendungen von Mikrofluidsystemen jedoch von entscheidender Bedeutung.

Ein Beispiel ist die Kultivierung von Zellen in einem Mikrokanal. Wachsen diese, so bilden sie toxische Stoffe, welche zu einem Absterben der Zellen führen. In engen Kanälen wird durch die geometrische Anordnung die Diffusion stark eingeschränkt, so dass die Konzentration der toxischen Stoffe im Bereich des Kanals schnell ansteigt. Findet kein Austausch der Lösung statt, ist ein zum Kanalinneren hin stark nachlassendes Zellwachstum zu beobachten. Eine konstante Versorgung mit Nährmedium und Entsorgung von toxischen Stoffen kann nur durch einen zeitlich regulierten Lösungswechsel in dem Kanal erfolgen. Pumpen haben dabei oft die oben beschriebenen Nachteile.

Aus den oben genannten Gründen ist es auch nicht möglich, Zellen in einem abgeschlossenen System zu transportieren oder über einen längeren Zeitraum, unter Beibehaltung ihres Stoffwechsels, zu lagern.

Eine weitere Anwendung von Pumpen ist das Aufreinigen von Flüssigkeiten. Hierzu gibt es unterschiedliche Methoden. Wichtig ist bei allen, dass erstens ein definierter Volumenstrom angelegt werden kann und zweitens den Reaktionspartnern (den in Lösung und der im Träger befindlichen Substanzen) bestimmten Reaktionszeiten zur Verfügung gestellt werden.

Eine weitere Anwendung ist der Nachweis von bestimmten Substanzen in einem Kanalsystem, bei denen es erforderlich ist, bestimmte Flüssigkeiten nacheinander durch denselben Kanal laufen zu lassen. Auch hier sind Flußraten und Reaktionszeiten einzuhalten. Pumpen müssten dabei extrem exakt arbeiten und über eine spezielle Elektronik gesteuert werden.

Aufgabe der Erfindung ist es, ein Mikrofluidsystem bereitzustellen, mit dem in einfacher Weise Flüssigkeiten transportiert werden können und ohne dass das Mikrofluidsystem einen externen Anschluß benötigt.

Diese Aufgabe wird gelöst durch das Mikrofluidsystem nach Anspruch 1. Dabei handelt es sich um ein Mikrofluidsystem mit einem Substrat, wenigstens zwei Reservoiren und wenigstens einem in das Substrat integrierten Kanal, der zwei Reservoire verbindet, wobei die Mündung wenigstens eines Kanals oberhalb der Grundfläche eines Reservoirs angeordnet ist.

Mit einem solchen Mikrofluidsystem können in einfacher Weise Flüssigkeiten transportiert werden, indem das Mikrofluidsystem nach einer Seite geneigt wird, wodurch eine Flüssigkeit von einem Reservoir (einem höher gelegenen Reservoir) durch einen damit verbundenen Kanal in ein am anderen Ende des Kanals angeordnetes Reservoir fließt. Die oberhalb der Grundfläche eines Reservoirs angeordnete Mündung des Kanals hat verschiedene Vorteile. Wenn eine Flüssigkeit durch diese Mündung in ein Reservoir fließt, wird dadurch verhindert, dass die Flüssigkeit wieder in den Kanal zurückfließen kann. Befindet sich die Flüssigkeit in einem Reservoir, in das ein Kanal mit einer oberhalb der Grundfläche angeordneten Mündung mündet, so kann fließt die Flüssigkeit erst dann aus dem Reservoir, wenn eine ausreichende Neigung des Mikrofluidsystems erreicht ist, so dass die Flüssigkeit in den Kanal eindringen kann. Auf diese Weise kann auch erreicht werden, dass nur eine vorherbestimmte Flüssigkeitsmenge aus einem befüllten Reservoir in einen Kanal fließt.

Das erfindungsgemäße Mikrofluidsystem kann eine Mehrzahl von Kanälen und/oder Reservoiren umfassen. Dabei können mehrere Kanäle in ein Reservoir münden. Vorteilhafterweise münden verschiedene Kanäle auf verschiedenen Höhen in ein Reservoir. Es können mehrere Reservoire mit unterschiedlichen Flüssigkeiten befüllt sein. Die Kanäle können sich vorzugsweise kreuzen und/oder teilen. Auf diese Weise können aus verschiedenen Reservoiren, ggf. je nach Neigung, verschiedene Flüssigkeiten in denselben Kanal fließen. Die Reservoire und Kanäle können vorteilhafterweise auch derart angeordnet sein, dass dadurch ein geschlossener Flüssigkeitskreislauf gebildet wird. Durch unterschiedliches Neigen des Mikrofluidsystems in verschiedene Richtungen, beispielsweise, kann eine Flüssigkeit, ausgehend von einem ersten Reservoir, abwechselnd durch Kanäle und Reservoire fließen, bis sie wieder im Ausgangsreservoir ankommt.

In einer bevorzugten Weiterbildung aller zuvor beschriebenen Mikrofluidsysteme kann die Grundfläche wenigstens eines Reservoirs relativ zu einer Reservoirseitenwand geneigt sein. Dies bedeutet, dass der Winkel zwischen der Grundfläche und der Seitenwand ungleich π/2 ist. Mit einer derart geeignet gewählten Reservoirgeometrie kann weiter eingestellt werden, bei welchem Neigungswinkel des Mikrofluidsystems eine Flüssigkeit aus einem Reservoir in einen Kanal fließt.

Gemäß einer vorteilhaften Weiterbildung kann das Mikrofluidsystem abgeschlossen sein und eine Vorrichtung zum Druckausgleich umfassen, die jedes Reservoir mit wenigstens einem anderen Reservoir verbindet. Damit kann insbesondere ein luftdichtes Abschließen ermöglicht werden. Durch das Abschließen des Mikrofluidsystems wird verhindert, dass die Flüssigkeit bzw. die Flüssigkeiten mit der Außenwelt in Kontakt kommen. Die Vorrichtung zum Druckausgleich kann in Form von in das Substrat integrierten Kanälen ausgebildet sein, die derart in die Reservoire münden, dass keine Flüssigkeit eindringen kann und sie somit nur dem Gas- bzw. Durckausgleich dienen. Die Vorrichtung kann vorzugsweise auch in Form von Leitungen ausgebildet sein, die die Reservoire verbinden, aber nicht in das Substrat integriert sind.

Gemäß einer vorteilhaften Alternative können die Reservoire mit gasdurchlässigen Deckeln abgeschlossen sein, so dass der Druckausgleich mit der Umgebung erfolgt.

Gemäß einer vorteilhaften Weiterbildung aller zuvor beschriebenen Mikrofluidsysteme kann wenigstens ein Reservoir mit einem Deckel verschlossen sein, wobei der Deckel einen Filter aufweist. Durch einen solchen Filter kann ein Gasaustausch mit der Umgebung oder mit einem anderen, verbundenen Reservoir ermöglicht werden, ohne dass Schadstoffe in das Reservoir eindringen oder aus dem Reservoir entweichen können.

In einer bevorzugten Weiterbildung aller zuvor beschriebenen Mikrofluidsysteme kann wenigstens ein Kanal eine geladene Oberfläche, Kolloide, Mikrofilter und/oder eine Einrichtung zur Rückflußverhinderung aufweisen.

Ist die Oberfläche z.b. positiv geladen, haften negativ geladene Organellen oder Moleküle an ihr. Die elektrostatische Kraft steigt mit der Ladung der Organelle oder des Moleküls an. Wird beispielsweise die Salzkonzentration im Kanal erhöht, sinkt die Debye-Länge sowie das Zetapotential ab (Gouy-Chapmann Theorie). Die weniger stark geladenen Organellen/Moleküle lösen sich ab und können z.b. durch Fluß abtransportiert werden. Die stark geladenen Organellen/Moleküle bleiben haften. So können Organellen/Moleküle nach ihrer Ladung getrennt werden. Somit können z.B. DNA-Moleküle aufgereinigt werden, wenn diese das am stärksten geladene Molekül bilden und deswegen am längsten (bei steigender lonenstärke) haften bleiben. Die DNA kann daraufhin mit bekannten Methoden analysiert werden.

In die Kanälen können Kolloide verschiedener Größe eingebracht sein. Diese können verschiedene Aufgaben erfüllen: das Aufreinigen von Flüssigkeiten; die Herausfilterung bestimmter Substanzen aus Flüssigkeiten; die Einbringung bestimmter Substanzen in Flüssigkeiten; das Einbringen bestimmter Substanzen bei Vorhandensein spezifischer Stoffe in der Flüssigkeit. Dabei kann ausgenutzt werden, dass Flüssigkeiten von verschiedenen Richtungen durch die Kolloidbarriere fließen können. Insbesondere können Kolloide mit einer funktionalisierten Oberfläche verwendet werden.

Mikrofilter können dazu dienen, bestimmte Stoffe herauszufiltern. In einer bevorzugten Weiterbildung können die Kolloide in Verbindung mit Mikrofiltern verwendet werden. Die Kolloide können auch durch eine Verjüngung eines Kanals in diesem fixiert werden; der Kanal kann bspw. konisch ausgebildet sein. Die Kolloide können z.B. durch Membranen mit einer definierten Porengröße oder Mikrofilter in dem Kanal fixiert werden. Es ist auch möglich, verschieden große Kolloide hintereinander anzuordnen.

Durch die Einrichtung zur Rückflußverhinderung wird ermöglicht, dass entsprechende Kanäle nur zum Flüssigkeitstransport in einer Kanalrichtung verwendet werden. Die Einrichtung zur Rückflußverhinderung kann vorzugsweise als Rückschlagventil ausgebildet sein.

In einer bevorzugten Weiterbildung aller zuvor beschriebenen Mikrofluidsysteme kann in einem Mikrofluidsystem wenigstens ein weiterer Kanal vorgesehen sein, der eine geladene Oberfläche, Feststoffe, insbesondere Kolloide, Mikrofilter und/oder eine Einrichtung zur Rückflußverhinderung aufweist. Somit können durch die verschiedenen Eigenschaften verschiedene Kanäle unterschiedliche Funktionen beispielsweise beim Analysieren von Zellen und/oder Zellkompartimenten übernehmen.

Gemäß einer vorteilhaften Weiterbildung aller zuvor beschriebenen Mikrofluidsysteme können diese optisch hoch transparente Bereiche und oder in das Substrat integrierte optische Komponenten aufweisen. Damit können Zellen und/oder Zellkompartimente in den Kanälen optisch untersucht werden.

Angesichts der eingangs diskutierten Nachteile ist eine weitere Aufgabe, ein System zum Transportieren von Flüssigkeiten bereitzustellen, in dem in einfacher Weise Flüssigkeiten transportiert werden können und ohne dass das Mikrofluidsystem einen externen Anschluß benötigt.

Diese Aufgabe wird gelöst durch ein System zum Transportieren von Flüssigkeiten in einem Mikrofluidsystem, umfassend ein Mikrofluidsystem mit einem Substrat, wenigstens zwei Reservoiren und wenigstens einem in das Substrat integrierten Kanal, der zwei Reservoire verbindet, und eine Vorrichtung zum Schwenken des Mikrofluidsystems um wenigstens eine Achse in wenigstens zwei Richtungen. Bei dem Mikrofluidsystem kann es sich vorteilhafterweise um eines der oben beschriebenen Mikrofluidsysteme handeln.

Dadurch ist die gezielte Bewegung von Flüssigkeiten in einem Mikrofluidsystem ohne externe Anschlüsse oder Verbindungen möglich. Nach Einbringen der zu untersuchenden Substanz in das Mikrofluidsystem kann dieses hermetisch abgeschlossen werden. Dies stellt eine deutliche Vereinfachung und Verbesserung bei Molekül- und Zelldiagnosen dar, da in einem solchen Träger Analysen insbesondere Langzeituntersuchungen ohne direkten Kontakt der zu untersuchenden Substanzen mit der Außenwelt durchgeführt werden können.

Unter Schwenken wird das Drehen um einen Winkel zwischen 0 und 2π verstanden. Das Mikrofluidsystem kann um eine Achse in zwei Richtungen und/oder um mehrere Achsen in eine Richtung geschwenkt werden. In einer vorteilhaften Weiterbildung kann das Mikrofluidsystem um eine Achse periodisch hin- und hergeschwenkt werden.

Durch die Neigung des Substrats kann die Flußrate eingestellt werden. Auf diese Weise können Flüssigkeiten in dem Mikrofluidsystem aufgrund von Gravitationskraft transportiert werden. Vorteilhafterweise ist die Vorrichtung zum Schwenken derart ausgebildet, dass das Mikrofluidsystem um mehrere Achsen geschwenkt werden kann. Diese Schwenkbewegungen um unterschiedliche Achsen können sich insbesondere überlagern; dabei kann eine Art taumelnde Bewegung des Mikrofluidsystems entstehen. Insbesondere kann eine Schwenkbewegung um einen Winkel kleiner als 2π um eine erste Achse mit einer vollständigen Kreisdrehung um eine zweite Achse überlagert werden. Bei einer Schwenkbewegung, insbesondere bei einer vollständigen Kreisdrehung, kann auch die Zentrifugalkraft, mit der die Flüssigkeiten beaufschlagt werden, für den Flüssigkeitstransport ausgenutzt werden.

Durch die Schwenkbewegung können Flüssigkeiten aus verschiedenen Richtungen, je nach Neigung des Trägers, durch ein und denselben Kanal strömen. Dies ist bei der Verwendung einer sich drehenden Unterlage, bei welcher die Flüssigkeiten ausschließlich durch die Zentrifugalkraft bewegt werden, nicht möglich. Auch ist eine zeitliche Abfolge der Strömungsrichtung und Strömungsgeschwindigkeit einfach und genau einzustellen. Dies ist besonders bei der kontrollierten Zusammenführung von mehreren Flüssigkeiten vorteilhaft.

Die Vorrichtung zum Schwenken kann beispielsweise hydraulische Einrichtungen umfassen, mit denen die Vorrichtung bewegt wird.

Vorzugsweise kann die Vorrichtung zum Schwenken eine Einrichtung zum Fixieren des Mikrofluidsystems aufweisen. Damit werden Mikrofluidsysteme immer an der gleichen Stelle der Vorrichtung angeordnet. Falls somit die Vorrichtung bestimmte Schwenkbewegungen automatisiert durchführt, können Untersuchungen mit hoher Genauigkeit wiederholt werden.

Erfindungsgemäß wird auch ein System zum Transportieren von Flüssigkeiten in einem Mikrofluidsystem bereitgestellt, umfassend ein Mikrofluidsystem mit einem Substrat, wenigstens zwei Reservoiren und wenigstens einem in das Substrat integrierten Kanal, der zwei Reservoire verbindet, wobei ein erstes Reservoir ein Volumen aufweist, und eine an dem ersten Reservoir angeordneten Vorrichtung, um das Volumen mit Druck zu beaufschlagen. Vorzugsweise handelt es sich bei dem Mikrofluidsystem um eines der zuvor beschriebenen Mikrofluidsysteme.

Vorzugsweise kann die Vorrichtung zur Druckbeaufschlagung als elastische Membran ausgebildet sein, die in Form eines Deckels auf dem Reservoir angeordnet ist. Durch mechanischen Druck auf die Membran wird das Volumen des Reservoirs mit Druck beaufschlagt.

Wie oben beschrieben, können die Mikrofluidsysteme eine Mehrzahl von Kanälen und/oder Reservoiren aufweisen. Dabei können mehrere Kanäle in ein Reservoir münden. Die Kanäle können sich auch kreuzen und/oder teilen.

Gemäß einer vorteilhaften Weiterbildung können die beiden zuvor beschriebenen Systeme zum Transportieren von Flüssigkeiten in einem Mikrofluidsystem miteinander kombiniert sein.

Die Erfindung stellt außerdem ein Verfahren zum Züchten und/oder Analysieren von Zellen mit den Schritten bereit: Bereitstellen eines Mikrofluidsystems mit einem Substrat, wenigstens zwei Reservoiren und wenigstens einem in das Substrat integrierten Kanal, der zwei Reservoire verbindet, Einbringen von wenigstens einer Zelle in wenigstens einen Kanal, Befüllen wenigstens eines Reservoirs mit einer Flüssigkeit, und Transportieren der Flüssigkeit durch einen mit dem befüllten Reservoir verbundenen Kanal durch Schwenken des Mikrofluidsystems um wenigstens eine Achse in wenigstens zwei Richtungen. Das Mikrofluidsystem kann um eine Achse in zwei Richtungen und/oder um mehrere Achsen in jeweils eine Richtung geschwenkt werden. Vorzugsweise kann es sich bei dem bereitgestellten Mikrofluidsystem um eines der zuvor beschriebenen Mikrofluidsysteme handeln.

Auf diese Weise können beispielsweise Zellen in einem Mikrofluidsystem gezüchtet werden, wobei die Nährmediumversorgung und die Produktentsorgung in das Mikrofluidsystem integriert sind. Durch ein periodisches Hin- und Herschwenken des Mikrofluidsystems kann beispielsweise Flüssigkeit von einem jeweils höher gelegenen Reservoir in ein tiefergelegenes Reservoir durch den Kanal strömen. Dabei können die Geometrien des Kanals und des Reservoirs so aufeinander abgestimmt sein, dass sich wesentlich mehr Flüssigkeit in den Reservoiren befindet als im Kanal (typischerweise ein Faktor 10-200). Bilden sich im Kanal toxische Stoffe, oder ändert sich die Konzentration des in der Flüssigkeit gelösten Gases im Kanal, kann diese Flüssigkeit durch Kippen des Trägers in eines der Reservoire geleitet werden. Aufgrund der hohen Flüssigkeitsmenge im Reservoir im Verhältnis zum Kanal findet dort eine entsprechende Verdünnung statt. Bei wiederholten Wippen wird somit die Anreicherung toxischer Stoffe und die Zuführung von Nährmedien im Kanal gewährleistet. Dadurch können Zellen über einen langen Zeitraum in einem Kanalsystem am Leben gehalten werden.

Erfindungsgemäß wird ein Verfahren zum Züchten und/oder Analysieren von Zellen mit den Schritten bereitgestellt: Bereitstellen eines Mikrofluidsystems mit einem Substrat, wenigstens zwei Reservoiren und wenigstens einem in das Substrat integrierten Kanal, der zwei Reservoire verbindet, Einbringen von wenigstens einer Zelle in wenigstens einen Kanal, Befüllen wenigstens eines Reservoirs mit einer Flüssigkeit, und Transportieren der Flüssigkeit durch einen mit dem befüllten Reservoir verbundenen Kanal durch Beaufschlagen der Flüssigkeit mit Druck. Vorzugsweise kann es sich bei dem bereitgestellten Mikrofluidsystem um eines der zuvor beschriebenen Mikrofluidsysteme handeln.

Gemäß einer vorteilhaften Weiterbildung können die zuvor beschriebenen Verfahren in einem Verfahren kombiniert werden.

Gemäß einer vorteilhaften Weiterbildung der zuvor beschriebenen Verfahren umfasst das Befüllen des Reservoirs weiterhin ein Befüllen mit einem Gas. Dadurch wird dafür gesorgt, dass in der Flüssigkeit eine bestimmte Konzentration eines bestimmten Gases oder eines Gasgemisches gelöst vorliegt. Die Konzentration des Gases in der Flüssigkeit kann dabei durch die Verweildauer der Flüssigkeit im Reservoir bestimmt werden. Eine konstante Versorgung mit gelösten Gasen in der Flüssigkeit kann durch eine Verbindung der Reservoirs mit dem entsprechenden Gas gewährleistet werden.

Durch das Befüllen eines Reservoirs, das mit einer Vorrichtung zur Druckbeaufschlagung versehen ist, kann erreicht werden, dass sich insbesondere oberhalb der Flüssigkeit ein Gasvolumen befindet. Damit kommt die Vorrichtung zur Druckbeaufschlagung nicht mit der Flüssigkeit in Kontakt und die Druckbeaufschlagung der Flüssigkeit erfolgt mittelbar über das Gasvolumen.

Gemäß einer vorteilhaften Weiterbildung der zuvor beschriebenen Verfahren wird ein Reservoir mit Feststoffen, insbesondere mit Kolloiden, und ein weiteres Reservoir mit einer Flüssigkeit zum Funktionalisieren der Feststoffe befüllt. Durch gezieltes Befüllen unterschiedlicher Reservoire und anschließendes Schwenken der Kammer können die so befüllten Reservoire mit Flüssigkeit gefüllt werden. Die in den Reservoiren befindlichen Feststoffe oder Teile der Feststoffe können sich somit in der Flüssigkeit lösen. Durch erneutes Schwenken kann die Flüssigkeit mit den gelösten Stoffen in einen ausgewählten Kanal geführt werden. Dies kann z.B. dazu dienen, Zellen in einer zeitlich definierten Abfolge mit Nährmedium zu versorgen. Auch kann so die Wirkung verschiedener Substanzen auf Zellen nachgewiesen werden.

Ein vorteilhaftes Verfahren zum Nachweis von Molekülen kann die Schritte umfassen: Moleküle in einer ersten Flüssigkeit können die Oberfläche eines Kolloids funktionalisieren. Moleküle einer zweiten Flüssigkeit binden an die funktionalen Gruppen der Kolloidoberfläche. Eine dritte Flüssigkeit enthält Moleküle, welche spezielle Komponenten der angebundenen und/oder funktionalen Gruppe mit angebundenem Molekül ablöst. In jedem dieser Teilschritte können bestimmte Reaktionen nachgewiesen werden.

Vorzugsweise können die Verfahren dazu verwendet werden, um Zellorganellen (beispielsweise Zellkern oder Mitochondrien) oder Zellmoleküle (DNA, Proteine) zu separieren und/oder zu identifizieren. Dazu werden die im Substrat/Kanal befindlichen Zellen zerstört. Dies kann z.B. durch osmotischen Druck, Scherkräfte, Druckluft, oder spezielle Lösungsmittel erfolgen. Die Anwendung dieser Methoden ist in einem Mikrofluidsystem besonders einfach zu realisieren. Die so freigesetzten Zellkompatimente können herausgefiltert, chemisch gebunden, im Gel separiert, elektrophoretisch bewegt und/oder getrennt, immobilisiert oder an geladenen Oberflächen fixiert werde. Die dazu nötigen Komponenten können in das Mikrofluidsystem eingebracht sein oder können in den Kanal gefüllt werden.

In Kombination mit zeitlich korrelierten Schwenkbewegungen können mit den oben beschriebenen Verfahren Organellen und/oder Moleküle in einem Mikrofluidsystem mit Kanälen, Reservoiren und den beschriebenen Komponenten vollkommen automatisiert aus Zellen separiert und analysiert werden.

In einer bevorzugten Weiterbildung können alle zuvor beschriebenen Verfahren derart durchgeführt sein, dass aufgrund der Schwenkbewegungen das Mikrofluidsystem ein selbstversorgendes System darstellt. Dies kann durch die Verwendung der oben dargestellten Ausführungen realisiert werden. Dadurch ist eine Langzeitversorgung der Zellen gewährleistet. Dies ermöglicht einen ,Sofort-Test' auf Zellbasis. Vorzugsweise ist der Träger zunächst durch einen Abziehdeckel abgeschlossen. Dieser wird bei Gebrauch geöffnet, so dass der Test durchgeführt werden kann. Dies ermöglicht einen lagerfähigen Test mit lebenden Zellen.

Gemäß einer bevorzugten Weiterbildung aller zuvor beschriebenen Verfahren umfassen diese den weiteren Schritt: Fluoreszenzanalyse der Zellen und/oder Zellkompartimente. Auf diese Weise kann das Züchten und Analysieren bis hin zur optischen Analyse in einem Mikrofluidsystem erfolgen.

Im folgenden werden Ausführungsbeispiele der Erfindung anhand einer Zeichnung weiter erläutert. Dabei zeigt:
Fig. 1 eine Querschnittsansicht eines Mikrofluidsystems mit einem Kanal und zwei Reservoiren,
Fig. 2 eine Querschnittsansicht eines Mirofluidsystems mit einer Vorrichtung zum Druckausgleich zwischen zwei Reservoiren,
Fig. 3 eine Querschnittsansicht eines Kanals mit darin angeordneten Kolloiden, und
Fig. 4 eine Querschnittsansicht eines Mikrofluidsystems mit zwei Reservoiren mit Kanalmündungen auf verschiedenen Höhen.

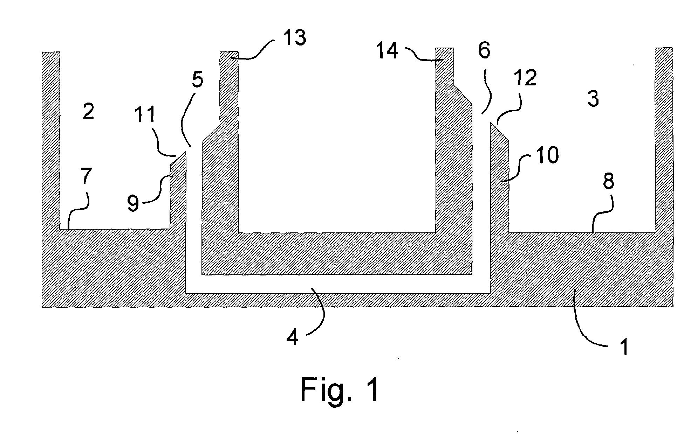

Fig. 1 zeigt ein Mikrofluidsystem mit einem Substrat 1, zwei Reservoiren 2 und 3 und einem in das Substrat 1 integrierten Kanal 4. Die Mündungen 5 und 6 des Kanals 4 sind jeweils oberhalb Grundflächen 7 und 8 der Reservoire 2 und 3 angeordnet. Innerhalb der Reservoire 2 und 3 sind auf der Grundfläche 7 und 8 stehende Hohlzylinder 9 und 10 ausgebildet, durch die der Kanal 4 geführt wird. Die Hohlzylinder 9 und 10 sind direkt entlang der Reservoirseitenwände 13 und 14 angeordnet und gehen in diese über. Die Mündungen 5 und 6 sind auf unterschiedlichen Höhen, von der Grundfläche 7 und 8 gesehen, angeordnet, so daß je nach Reservoir ein unterschiedlicher Neigungswinkel des Mikrofluidsystems erforderlich ist, damit eine Flüssigkeit in den Kanal 4 fließt. Die mündungsseitigen Oberflächen 11 und 12 der Hohlzylinder 9 und 10 sind abgeschrägt, damit die Flüssigkeit beim Ausströmen aus der Mündung nicht nach oben spritzt sonder zur Seite fließt.

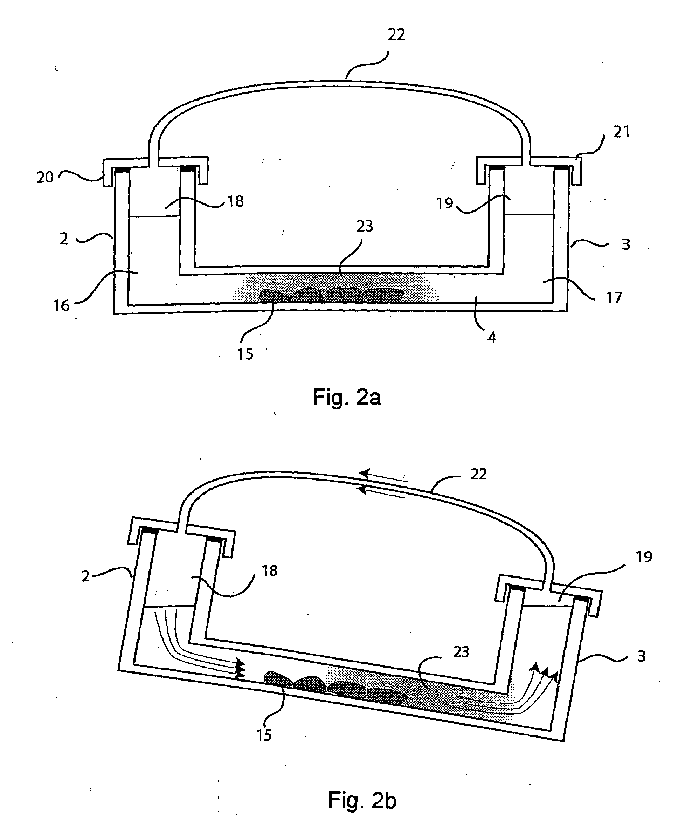

Fig. 2a zeigt ein Mikrofluidsystem, wobei in dem Kanal 4 Zellen 15 angeordnet sind. Die Flüssigkeitsreservoire 2 und 3 umfassen jeweils Flüssigkeitsvolumina 16 und 17 und - darüber angeordnet - Gasvolumina 18 und 19. Bei der Flüssigkeit handelt es sich um ein Nährmedium. Die Reservoire 2 und 3 sind durch Deckel 20 und 21 luftdicht abgeschlossen abgeschlossen. Die Reservoire 2 und 3 sind durch die Deckel über einem Gaskanal 22 miteinander verbunden, wodurch ein Gasaustausch und somit ein Druckausgleich ermöglicht wird. Während der Kultivierung der Zellen 15 entstehen toxische Stoffe 23.

Um die toxischen Stoffe zu verdünnen bzw. von den Zellen zu entfernen und gleichzeitig Nährmedium zuzuführen, wird das Mikrofluidsystem nach einer Seite geschwenkt. Ein derart geneigtes Mikrofluidsystem ist in Fig. 2b zu sehen. Durch die Neigung strömt Flüssigkeit vom Reservoir 2 in das niedriger gelegene Reservoir 3. Die toxischen Stoffe 23 werden von den Zellen 15 entfernt und in dem Reservoir 3 verdünnt; außerdem wird den Zellen frisches Nährmedium aus dem Reservoir 2 zugeführt. Das Gas aus dem verkleinerten Gasvolumen 19 strömt über den Gaskanal 22 in das Reservoir 2, wodurch ein Druckausgleich geschaffen wird.

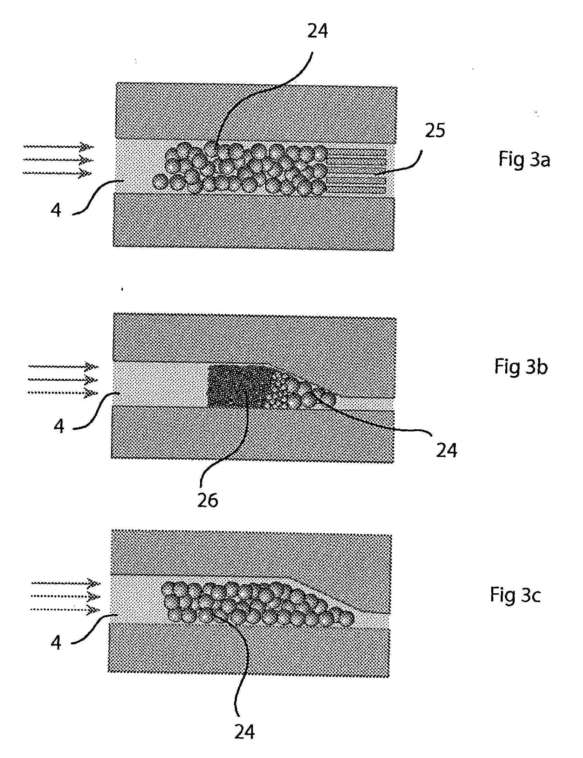

In Fig. 3 werden verschiedene Kolloidanordnungen in einem Kanal 4 gezeigt. In Fig. 3a umfasst der Kanal Kolloide 24, die durch einen Mikrofilter 25 fixiert sind. In Fig. 3b verjüngt sich der Kanal 4 in Flußrichtung. Vor dier Verengung lagern sich große Kolloide 24 an. Dahinter sind dann kleinere Kolloide 26 angeordnet, wodurch eine Kolloidbarriere aus unterschiedlich großen Kolloiden gebildet wird. Die Kolloidbarriere in Fig. 3c umfasst nur große Kolloide 24, die vor einer Verengung fixiert werden.

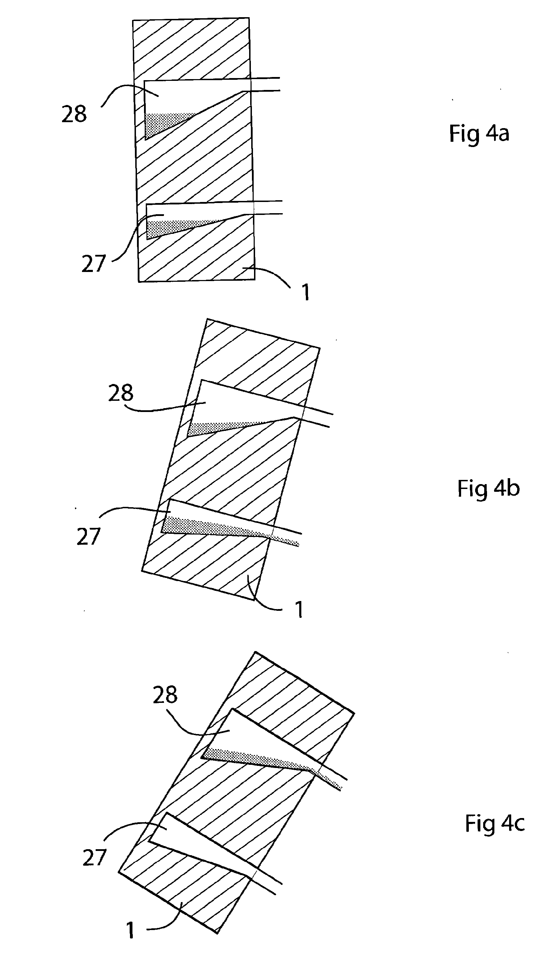

Fig. 4a zeigt einen Querschnitt eines Mikrofluidsystems mit einem Substrat 1, in welchem zwei Reservoire 27 und 28 übereinander angeordnet sind. Die Reservoire können von der Seite her zugänglich sein, indem seitlich jeweils ein weiterer Kanal (nicht gezeigt) in jedes Reservoir mündet. Die Grundflächen der Reservoire sind relativ zur Seitenfläche geneigt, wobei der Neigungswinkel unterschiedlich ist. Wird das Mikrofluidsystem um einen ersten, kleineren Winkel geschwenkt (Fig. 4b), so erreicht nur dir Flüssigkeit in dem unteren Reservoir 27 die Kanalmündung und fließt in den Kanal. Bei einem stärkeren Neigungswinkel des Mikrofluidsystems (Fig. 4c) erreicht die Flüssigkeit aus dem zweiten Reservoir 28 die Kanalmündung und kann diesen Kanal durchströmen.

## Patentansprüche

1. Mikrofluidsystem mit einem Substrat, wenigstens zwei Reservoiren und wenigstens einem in das Substrat integrierten Kanal, der zwei Reservoire verbindet, wobei die Mündung wenigstens eines Kanals oberhalb der Grundfläche eines Reservoirs angeordnet ist.

2. Mikrofluidsystem nach Anspruch 1, wobei das Mikrofluidsystem abgeschlossen ist und eine Vorrichtung zum Druckausgleich umfasst, die jedes Reservoir mit wenigstens einem anderen Reservoir verbindet.

3. Mikrofluidsystem nach Anspruch 1 oder 2, in welchem wenigstens ein Kanal eine geladene Oberfläche, Feststoffe, insbesondere Kolloide, Mikrofilter und/oder eine Einrichtung zur Rückflußverhinderung aufweist.

4. Mikrofluidsystem nach einem der vorangegangenen Ansprüche, in welchem wenigstens ein weiterer Kanal vorgesehen ist, der eine geladene Oberfläche, Feststoffe, insbesondere Kolloide, Mikrofilter und/oder eine Einrichtung zur Rückflußverhinderung aufweist.

5. System zum Transportieren von Flüssigkeiten in einem Mikrofluidsystem umfassend
ein Mikrofluidsystem, insbesondere nach einem der vorangegangenen Ansprüche, mit einem Substrat, wenigstens zwei Reservoiren und wenigstens einem in das Substrat integrierten Kanal, der zwei Reservoire verbindet, und
eine Vorrichtung zum Schwenken des Mikrofluidsystems um wenigstens eine Achse in wenigstens zwei Richtungen.

6. System zum Transportieren von Flüssigkeiten in einem Mikrofluidsystem umfassend
ein Mikrofluidsystem, insbesondere nach einem der Ansprüche 1 - 4, mit einem Substrat, wenigstens zwei Reservoiren und wenigstens einem in das Substrat integrierten Kanal, der zwei Reservoire verbindet, wobei ein erstes Reservoir ein Volumen aufweist, und
eine an dem ersten Reservoir angeordneten Vorrichtung, um das Volumen mit Druck zu beaufschlagen.

7. Verfahren zum Züchten und/oder Analysieren von Zellen mit den Schritten:
Bereitstellen eines Mikrofluidsystems, insbesondere nach einem der Ansprüche 1 - 4, mit einem Substrat, wenigstens zwei Reservoiren und wenigstens einem in das Substrat integrierten Kanal, der zwei Reservoire verbindet,
Einbringen von wenigstens einer Zelle in wenigstens einen Kanal,
Befüllen wenigstens eines Reservoirs mit einer Flüssigkeit, und
Transportieren der Flüssigkeit durch einen mit dem befüllten Reservoir verbundenen Kanal durch Schwenken des Mikrofluidsystems um wenigstens eine Achse in wenigstens zwei Richtungen.

8. Verfahren zum Züchten und/oder Analysieren von Zellen mit den Schritten:
Bereitstellen eines Mikrofluidsystems, insbesondere nach einem der Ansprüche 1 - 4, mit einem Substrat, wenigstens zwei Reservoiren und wenigstens einem in das Substrat integrierten Kanal, der zwei Reservoire verbindet,
Einbringen von wenigstens einer Zelle in wenigstens einen Kanal,
Befüllen wenigstens eines Reservoirs mit einer Flüssigkeit, und
Transportieren der Flüssigkeit durch einen mit dem befüllten Reservoir verbundenen Kanal durch Beaufschlagen der Flüssigkeit mit Druck.

9. Verfahren nach Anspruch 7 oder 8, wobei das Befüllen des Reservoirs weiterhin ein Befüllen mit einem Gas umfasst.

10. Verfahren nach einem der Ansprüche 7 - 9, wobei ein Reservoir mit einem Lösungsmittel zum Zerstören von Zellen und/oder ein Reservoir mit einer Flüssigkeit zum Aufreinigen von Molekülen befüllt wird.

11. Verfahren nach einem der Ansprüche 7 - 10, wobei ein Reservoir mit Feststoffen, insbesondere Kolloiden, und ein weiteres Reservoir mit einer Flüssigkeit zum Funktionalisieren der Feststoffe befüllt wird.

12. Verfahren nach ein der Ansprüche 7 - 11 mit dem weiteren Schritt:
Fluoreszenzanalyse der Zellen und/oder Zellkompartimente.
